Europäisches Patentamt

European Patent Office

Office européen des brevets

(19)

(11) Publication number: **0 083 847**
**A2**

# (12) EUROPEAN PATENT APPLICATION

(21) Application number: 82306506.5

(22) Date of filing: 07.12.82

(51) Int. Cl.³: **A 61 D 17/00**

(30) Priority: 11.12.81 GB 8137419
26.02.82 GB 8205800

(43) Date of publication of application:
20.07.83 Bulletin 83/29

(84) Designated Contracting States:
AT BE CH DE FR GB IT LI LU NL SE

(71) Applicant: HRH INDUSTRIES & TRADING LIMITED
Stadle 36
FL-9490 Vaduz(LI)

(72) Inventor: Metcalf, Peter Edward Anthony
Jubilee House Topcliffe
Nr. Thirsk North Yorkshire(GB)

(74) Representative: Wharton, Peter Robert et al,
URQUHART-DYKES & LORD Beckett's Bank Chambers
19 Cheapside
Bradford BD1 4HR(GB)

(54) Respirator for animals associated with analysing means.

(57) A respirator for horses comprises a face mask 12 adapted to fit in substantially air tight relationship over the nose and muzzle of a horse. The mask has inlet and outlet pipes 14, 16 connected respectively to an air supply 18 and air collection unit 24. The latter has associated means for analysing the collected air. A reservoir of cleaning fluid and an associated compressor is provided to flush out and disinfect the apparatus between successive uses.

FIG.1.

EP 0 083 847 A2

Croydon Printing Company Ltd

- 1 -

RESPIRATOR

This invention relates to a respirator, especially to a respirator for use with horses.

In assessing the physiological capability of horses, in particular race horses, one of the measures made is the oxygen uptake of the horse under conditions of exertion. In order to do this it has been proposed to provide a sealed environmentally controlled chamber within which the horse is exercised to a certain point of exertion as determined by the horse's pulse rate, and measuring the oxygen depletion of the air within the chamber. It will be appreciated that the provision of such a sealed environmentally controlled chamber is expensive and the measurement of oxygen depletion in such a large amount of air is necessarily difficult. Furthermore, before the chamber can be used for a subsequent horse it must be completely flushed out so as to remove any residence of carbon dioxide or other body emissions, and disinfected so as to reduce the risk of infection. This process takes a considerable period of time and so limits the number of horses which can be processed in such a chamber in any given period of time.

The invention seeks to overcome the above mentioned disadvantages by the provision of a simple and relatively inexpensive respirator which may be flushed out and sterilised  ready for a subsequent horse within a few minutes.

According to the present invention there is provided a respirator which comprises a face mask adapted to fit in substantially air-tight relationship over the nose and muzzle of a horse, the mask having air inlet and outlet pipes connected respectively to an air supply and air collection unit, the latter having associated means for analysing the collected air.

The face mask ensures that the air breathed by the horse is received entirely from the air supply and exhaled exclusively to the collection unit. The air supply may comprise a reservoir of air which has been sterilised and adjusted to the desired temperature and humidity. A flow meter may be provided to measure the flow of air to the horse, and a pressure gauge and pressure adjusting means may also be provided. Preferably the air pressure is adjusted so that at the face mask the pressure experienced by the horse is exactly ambient atmospheric pressure.

The collection unit may comprise a tank for receiving the expended air and associated means, known in themselves, for analysing the expended air to determine the oxygen uptake of the animal under test.

A reservoir of cleaning fluid may be provided which can be flushed throughout the entire system in between successive animals under test and, owing to the relatively small volume of the system, the clean flush time is typically in the order of a few minutes. If desired, the supply and collection units can be made symmetrical so that, by operation of a suitable flow valve the unit can be operated in either direction.

Where an environmentally controlled, or at least a separately air conditioned chamber is available, the respirator of the invention may be used in a simplified fashion. Instead of the air supply to the respirator coming from a separate reservoir, ambient air from the chamber may be drawn in through the air inlet of the respirator. The latter should nevertheless preferably be fitted with an air flow meter as before. The exhaled air may be led to a collection unit as before where it is analysed. This form of operation, known as an open spirometric system, depends on the ambient air within the chamber being capable of rapid change, particularly between horses and it is preferred that a chamber should be provided with vacuum pumps to set out contaminated air and draw in fresh conditioned air between each animal under test.

Owing to advances in miniaturisation, it is now possible to obtain oxygen and $CO_2$ analysing equipment, as well as flow meters, of sufficiently small size that they can be incorporated into the respirator itself. Such equipment would preferrably have a direct read out, for example a digital read out, and the incorporation of the analysing and flow rate measuring equipment into the respirator is within the ambit of the invention.

- 4 -

The invention will be described further, by way of example, with reference to the accompanying drawings, in which:

Figure 1 is a diagrammatic view of a system in accordance with the invention; and

Figure 2 is a diagrammatic view of another embodiment.

Referring to the drawings, it can be seen that an animal under test, in this case a race horse 10, is fitted with a face mask 12 which may be lined with adhesive quality stretch rubber to give an air-tight seal about the muzzle and nose of the animal 10. The face mask 12 is provided with inlet and outlet pipes 14 and 16 respectively. The inlet pipe 14 delivers sterile air at a predetermined humidity and temperature to the mask from a supply reservoir 18 fed from an air intake 20 via sterilisation and filtration apparatus 22. Similarly, spent air is fed through the outlet pipe 16 to a collection unit which comprises a collection reservoir or tank 24 with an associated two-way compressor 26 and exhaust 28.

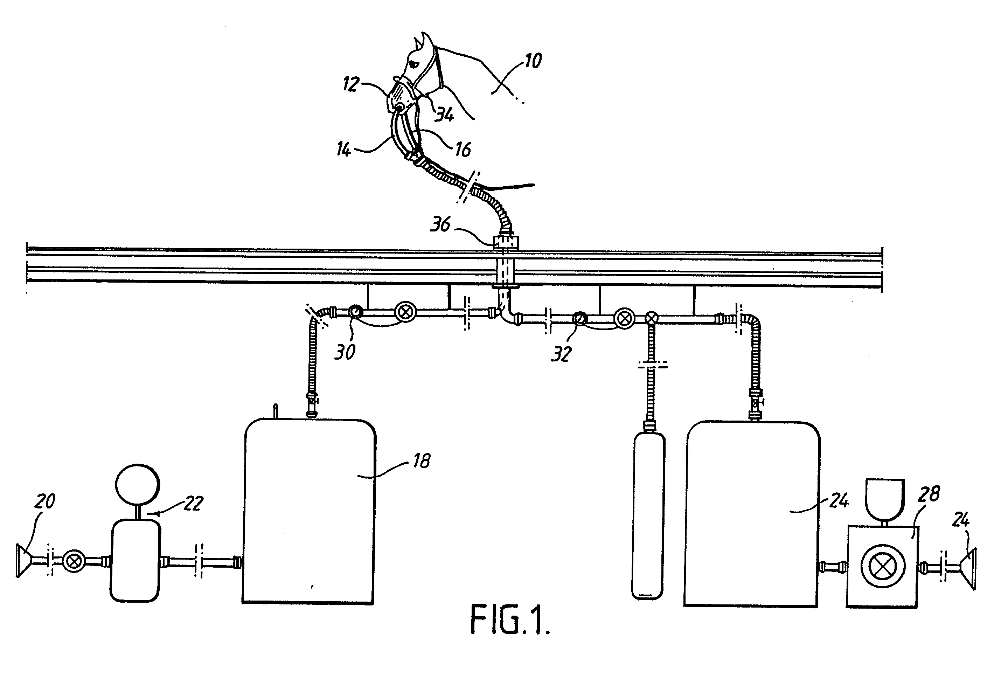

As illustrated in Figure 1, the supply and collection units are located beneath the floor of a suitable chamber the inlet and exit pipes 14, 16 passing through the floor to the face mask 12 fitted to the horse 10. The length of the inlet and exhaust pipes should be sufficient to allow the horse considerable movement so that it may be exercised to a given level of exertion without being restricted by the presence of pipes. Flow meters and pressure gauges 30 and 32 are provided to monitor the pressure of the air reaching the horse which should ideally be adjusted to exactly match the ambient air temperature, and the flow rate of the air being respired.

In use, the horse is led into the chamber and fitted with the face mask 12. The horse is then led through exercises to bring its pulse rate up to the rate desired. This could be maximum exertion, for example about 240 beats per minute, or. a lesser rate of, for example, about 160 beats per minute. In order to monitor this, a transistorised heart monitor 34 is provided at a convenient point, for example on the underside of the face mask 12. The oxygen usage on the horse at this stage is then measured as already described, from which an assessment of the physiological capabilities of the horse can be made. In order to allow the horse to have free movement within the chamber the inlet and exit pipes 14, 16 pass through the floor at a swivle valve 36 allowing free rotation of the pipes.

It will be appreciated, that while the invention is particularly suitable for horses, especially race horses, it may apply to other animals where an accurate assessment of respiration rate is required. Naturally, the contours of the face mask would be adjusted accordingly.

An especially useful application to which the respirator of the invention may be applied, the oxygen uptake rate of a horse may be measured some time after it has been connected to the apparatus and allowed to rest and become quiescent. The horse may then be taken up to maximum exertion and the oxygen uptake measured after the exertion has stopped until the horse's respiration and pulse rate are back to the normal quiescent state. The difference in the oxygen uptake between that recorded over this period and the oxygen uptake during the like period when the horse is quiescent, gives a measure of the "oxygen debt" which the horse builds up during heavy exertion. This quantity is a useful indication of the ability of the horse to accelerate and produce bursts of sustained high speed.

- 6 . -

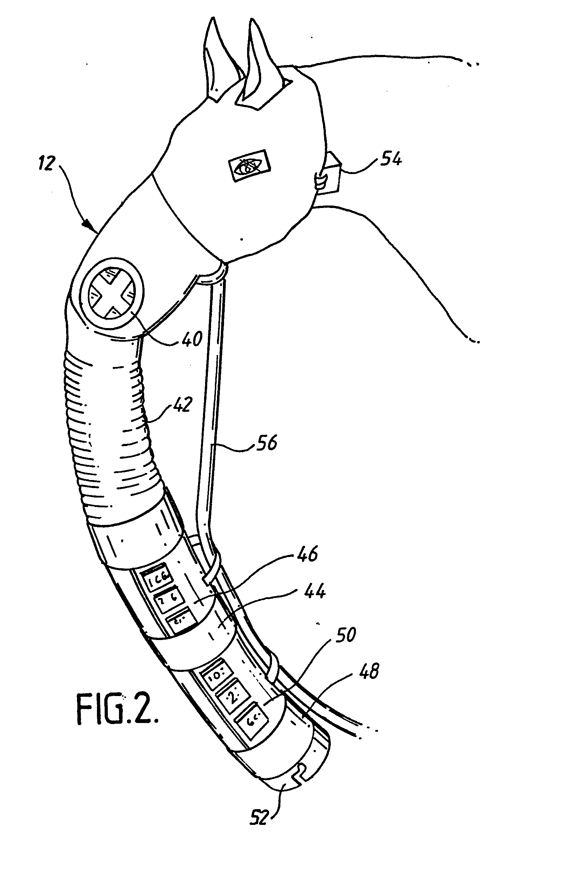

Referring now to figure 2, in this case the face mask 12 has a one-way inlet valve 40 and an outlet hose 42. The latter houses an oxygen analyser 44 with a digital telemetric read out and timer 46; and a $CO_2$ analyser 48 with a digitalised telemetric read out and timer 50. The end 52 of the hose 42 may be left open or connected to further tubing as necessary. The horse is also fitted with a telemetric pulse monitor 54, and a lunge line 56 may be attached. This form of the respirator of the invention is extremely compact and may be used where the ambient atmosphere is suitable for direct input into the respirator.

CLAIMS:-

1. A respirator which comprises a face mask adapted to fit in substantially airtight relationship over the nose and muzzle of a horse, the mask having air inlet and outlet pipes connected respectively to an air supply and air collection unit, the latter having associated means for analysing the collected air.

2. Apparatus as claimed in claim 1 in which the air supply comprises a reservoir of air which has been sterilised and adjusted to a desired temperature and humidity.

3. Apparatus as claimed in either of claim 1 or claim 2 further including a flow meter to measure the flow of air to the horse and a pressure gauge and pressure adjusting means for regulating the air pressure.

4. An apparatus as claimed in any one of claims 1 to 3 in which the collection unit comprises a tank for receiving the spent air.

5. An apparatus as claimed in any one of claims 1 to 4 in which a reservoir of cleaning fluid is provided in association with pump means for cleaning and disinfecting the apparatus.

6. An apparatus as claimed in claim 1 including a flow meter to measure the flow of air to the horse and in which the air collection unit comprises analysing means located on the respirator itself in communication with the outlet thereof.

FIG.1.

FIG.2.